# EUROPEAN PATENT SPECIFICATION

(11) **EP 0 712 411 B1**
(45) Date of publication and mention of the grant of the patent: **11.06.1997**
(21) Application number: 94924798.5
(22) Date of filing: 22.07.1994
(51) Int. Cl.: C07H 17/08, A61K 31/70, A01N 43/90

(54) **ANTIPARASITIC AGENTS**
ANTIPARASITICHES MITTEL
AGENTS ANTIPARASITAIRES

(30) Priority: 04.08.1993 GB 9316129
(43) Date of publication of application: 22.05.1996
(73) Proprietor: Pfizer Limited, Sandwich Kent CT13 9NJ (GB); PFIZER INC., New York, N.Y. 10017 (US)
(72) Inventor: DUTTON, Christopher, James, Sandwich, Kent CT13 9NJ (GB); GIBSON, Stephen, Paul, Sandwich, Kent CT13 9NJ (GB); WITTY, Michael, John, Sandwich, Kent CT13 9NJ (GB)
(74) Representative: Hayles, James Richard
(86) International application number: PCT/EP94/02433
(87) International publication number: WO 95/04746

(56) References cited:
- EP-A- 0 317 148
- EP-A- 0 350 187
- PATENT ABSTRACTS OF JAPAN vol. 12, no. 132 (C-490) 22 April 1988 & JP,A,62 252 788 (SANKYO CO LTD) 4 November 1987

## Description

This invention relates to antiparasitic agents and in particular to compounds related to the avermectins and milbemycins but having an alkylidene substituent at the 5-position.

The avermectins are a group of broad-spectrum antiparasitic agents referred to previously as the C-076 compounds. They are produced by fermenting a certain strain of microorganism Streptomyces avermitilis in an aqueous nutrient medium. The preparation and structure of these compounds obtained by fermentation are described in British Patent Specification 1573955. The milbemycins are structurally related macrolide antibiotics lacking the sugar residues at the 13-position. They may be produced by fermentation, for example as described in British Patent Specification No. 1390336 and European Patent Specification No. 0170006.

Compounds related to the original C-076 avermectins have also been prepared by fermentation of avermectin-producing microorganisms. For example European Patent Specifications 0214731 and 0317148 describe production of compounds related to the C-076 avermectins but having a different substituent at the 25-position by fermentation in the presence, in the fermentation medium, of certain acids.

In addition to these fermentation-derived products, a large number of publications describe compounds derived semisynthetically from these products, many of which possess useful antiparasitic properties. Some of this chemistry is reviewed in Macrolide Antibiotics, Omura S., Ed., Academic Press, New York (1984) and by Davies, H.G. and Green, R.H. in Natural Product Reports (1986), 3, 87-121 and in Chem Soc Rev (1991), 20, 211-269 and 271-239.

Other publications mentioning different combinations of substituents at various positions on the avermectin or milbemycin nucleus are EP-A-317148, 340932, 355541, 350187, 410165, 259779 and 254583; DE-A-2329486 and GB-A-2166436.

No avermectin derivatives having an alkylidene substituent at the 5-position are known, neither has any process capable of producing such compounds been reported. Japanese Patent Application No. 86-94754 (published under No. 87-252788) of Sankyo describes milbemycins and aglycone derivatives having a methylidene substituent at the 5-position, but the processes described for making them cannot be used for making similarly substituted avermectins or avermectin monosaccharides, as saccharide groups are removed by hydrolysis under the acidic conditions used.

It has now been discovered that avermectin derivatives and their monosaccharides having an alkylidene substituent at the 5-position may be prepared and that certain of these compounds have unexpected antiparasitic properties, in particular high potency against important arthropod parasites of cats and dogs. In addition, they have improved safety in mammals compared to previously known avermectins.

According to one aspect of the invention, there are provided compounds of formula (I) having antiparasitic activity 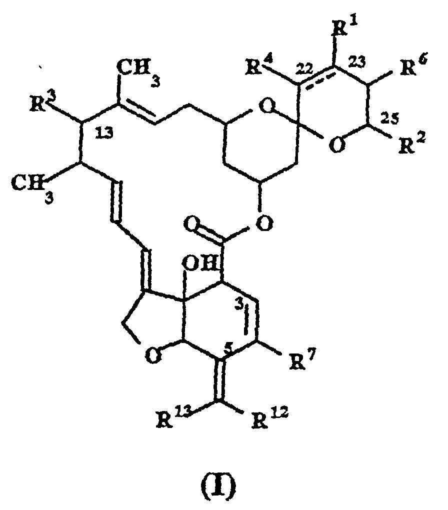 wherein the broken line represents an optional bond, R¹ and R⁴ being absent when this bond is present, R¹ and R⁴ are independently H or OR¹⁴ where R¹⁴ is H, C₁-C₈ alkyl, C₂-C₈ alkenyl, aralkyl, C₂-C₈ alkanoyl, C₃-C₈ alkenoyl, aralkanoyl, aroyl or carbamoyl;
R² is:
   (a) an alpha-branched C₃-C₈ alkyl, alkenyl, alkoxy-alkyl, or alkylthioalkyl group; an alpha-branched C₄-C₈ alkynyl group; a (C₅-C₈)cycloalkyl-alkyl group wherein the alkyl group is an alpha-branched C₂-C₅ alkyl group; a C₃-C₈ cycloalkyl or C₅-C₈ cycloalkenyl group, either of which may optionally be substituted by methylene or one or more C₁-C₄ alkyl groups or halo atoms; or a 3 to 6 membered oxygen or sulphur containing heterocyclic ring which may be saturated, or fully or partially unsaturated and which may optionally be substituted by one or more C₁-C₄ alkyl groups or halo atoms; or
   (b) a group of the formula -CH₂R⁸ wherein R⁸ is H, C₁-C₈ alkyl, C₂-C₈ alkenyl, C₂-C₈ alkynyl, alkoxyalkyl or alkylthioalkyl containing from 1 to 6 carbon atoms in each alkyl or alkoxy group, wherein any of said alkyl, alkoxy, alkenyl or alkynyl groups may be substituted by one or more halo atoms; or R⁸ is a C₃-C₈ cycloalkyl or C₅-C₈ cycloalkenyl group, either of which may optionally be substituted by methylene or one or more C₁-C₄ alkyl groups or halo atoms; or R⁸ is a 3 to 6 membered oxygen or sulphur containing heterocyclic ring which may be saturated, or fully or partially unsaturated and which may optionally be substituted by one or more C₁-C₄ alkyl groups or halo atoms; or R⁸ is a group of the formula SR⁹ wherein R⁹ is C₁-C₈ alkyl, C₂-C₈ alkenyl, C₃-C₈ alkynyl, C₃-C₈ Cycloalkyl, C₅-C₈ cycloalkenyl, phenyl or substituted phenyl wherein the substituent is C₁-C₄ alkyl, C₁-C₄ alkoxy or halo; or R⁸ is a 3 to 6 membered oxygen or sulphur containing heterocyclic ring which may be saturated, or fully or partially unsaturated and which may optionally be substituted by one or more C₁-C₄ alkyl groups or halo atoms; or
   (c) a C₁-C₆ alkyl group substituted by one oxo or one or more hydroxy groups or by a single oxygen atom on two adjacent carbon atoms forming an oxirane ring, or R² is a C₁-C₅ alkyl group substituted by a (C₁-C₆) alkoxycarbonyl group, said substituents on R₂ being attached to either or both of a terminal carbon atom and a carbon atom adjacent a terminal carbon atom of R²; or
   (d) = CH₂ or a group of the formula: 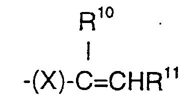 wherein R¹⁰ and R¹¹ are both H; R¹⁰ is H and R¹¹ is C₁-C₃ alkyl, or one of R¹⁰ and R¹¹ is H and the other is phenyl, heteroaryl, C₂-C₆ alkoxycarbonyl or substituted phenyl or heteroaryl wherein said substituent is fluorine, chlorine, C₁-C₄ alkyl, C₁-C₄ alkoxy, C₁-C₄ alkylthio, hydroxy(C₁-C₄)alkyl, cyano, aminosulphonyl, C₂-C₆ alkanoyl, C₂-C₆ alkoxycarbonyl, nitro, trifluoromethyl, trifluoromethoxy, amino or mono or di(C₁-C₄) alkylamino; and X is a direct bond or is an alkylene group having from 2 to 6 carbon atoms which may be straight or branched-chain; or
   (e) phenyl which may optionally be substituted with at least one substituent selected from C₁-C₄ alkyl, C₁-C₄ alkylthio groups, halo atoms, trifluoromethyl, and cyano;
or R² may be a group of formula (II): 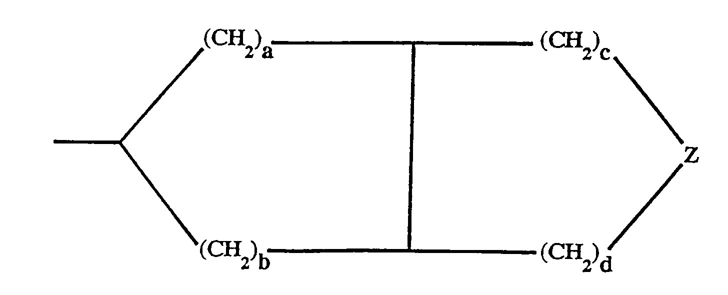 wherein Z is O, S or -CH₂- and a, b, c and d may each independently be 0,1 or 2, the sum of a, b, c and d not exceeding 5;
R⁶ is H or C₁-C₆ alkyl;
R⁷ is CH₃, -CH₂-OR¹⁴ where R¹⁴ is as defined above, or -CH₂X where X is a halogen atom; and
R³ is a group of formula: 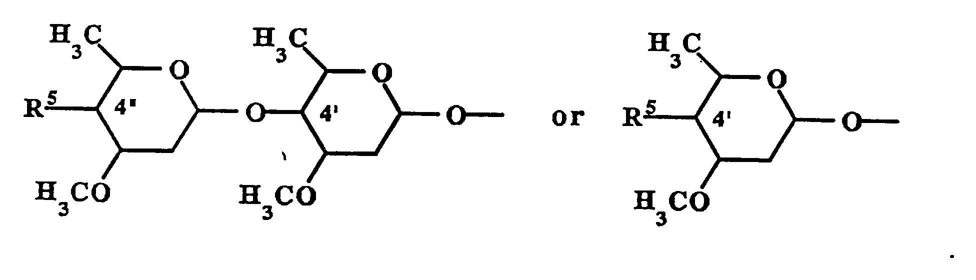 wherein R⁵ is attached to C-4" or C-4' by a single bond and is hydrogen, halo, hydroxy, C₁-C₉ alkanoyloxy or alkenoyloxy, aroyloxy, C₁-C₈ alkoxy, amino, N-(C₁-C₈)alkylamino, N,N-di(C₁-C₉)-alkylamino, N-(C₁-C₉)alkanoylamino, or N,N-di(C₁-C₉)alkanoylamino;
or R⁵ is attached to C-4" or C-4' by a double bond and is oxo, optionally substituted oximino, semicarbazido, N-(C₁-C₄)alkylsemicarbazono, N,N-di(C₁-C₄)alkylsemicarbazono, (C₁-C₄)alkylbenzoylhydrazono, and R¹² and R¹³ are independently H, CN, CONH₂, C₁-C₆ alkyl or aryl optionally substituted with at least one halo, OH, C₁-C₆ alkoxy or C₁-C₆ alkylthio group.

In all the above definitions, unless the context requires otherwise, alkyl groups containing 3 or more carbon atoms may be straight or branched-chain; halo means fluoro, chloro, bromo or iodo; and aryl means phenyl optionally substituted by one or more C₁-C₄ alkoxy groups or halo atoms.

Compounds within the scope of the invention include
5-cyanomethylidene-25-cyclohexyl avermectin B2;
5-carbamoylmethylidene-25-cyclohexyl avermectin B2;
5-cyanomethylidene-22,23-dihydroavermectin B1a monosaccharide;
5-methylidene-22,23-dihydroavermectin B1a monosaccharide;
5-methylidene-25-cyclohexyl-22,23-dihydroavermectin B1 monosaccharide;
5-methylidene-25-cyclohexylavermectin B2;
and 5-ethylidene-25-cyclohexylavermectin B2.

The compounds of formula (I) may be prepared from a compound of formula (II): 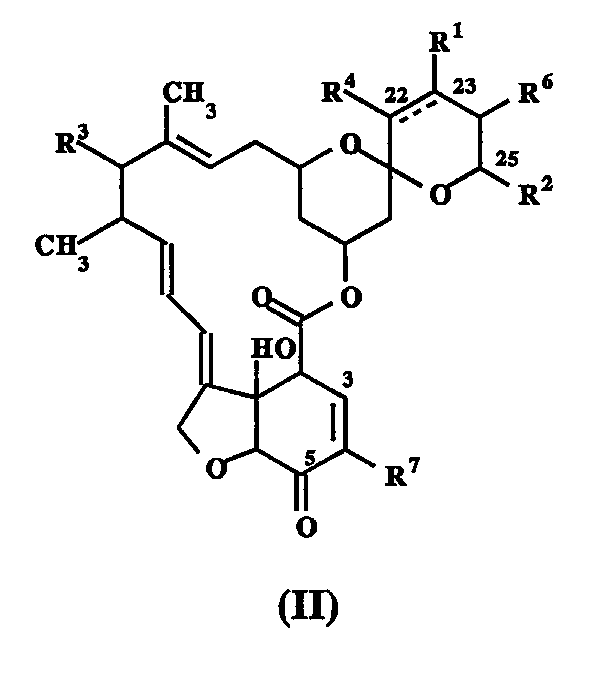 wherein R¹, R², R³, R⁴, R⁶ and R⁷ are as defined above. The compounds of formula (II) may generally be prepared by oxidation of the corresponding 5-hydroxy compound, for example using manganese dioxide, groups R¹-R⁷ being protected by conventional methods if necessary. These 5-hydroxy analogues may themselves be prepared by methods known in the art.

The addition of a phosphorus ylide of the formula R¹³R¹²C=PPh₃ to a compound of the formula (II) at low temperature (-100°C to 0°C) in an inert organic solvent such as tetrahydrofuran produces a compound of formula (I). The phosphorus ylide may be prepared using known methods from a compound of formula R¹³R¹²CH⊕PPh₃X⊖, where x⊖ is a halide ion, in the presence of base. Alternatively a compound of formula R¹³R¹²CHP(O)(OR¹⁵)₂, where R¹⁵ is an alkyl group, may firstly be treated with a base, then added to a compound of formula (II), to produce a compound of formula (I). This reaction may also be performed in a two-phase mixture of a chlorinated organic solvent and aqueous alkali containing a compound of formula R¹³R¹²CHP(O)(OR¹⁵)₂ and a phase-transfer reagent and a compound of formula (II), to form a compound of formula (I).

When it is desired to prepare a compound in which R¹² or R¹³ is carbamoyl, a phosphorus ylide in which R¹² or R¹³ is cyano may be used to obtain a cyanomethylidene derivative of formula (I) which may be hydrolysed under mild conditions, for example in the presence of manganese dioxide in methylene chloride at ambient temperature.

The compounds of the invention are effective in treating a variety of conditions caused by endoparasites including, in particular, helminthiasis which is most frequently caused by a group of parasitic worms described as nematodes and which can cause severe economic losses in swine, sheep, horses and cattle as well as affecting domestic animals and poultry. The compounds are also effective against other nematodes which affect humans and various species of animals including, for example, Dirofilaria in dogs and various parasites which can infect animals and humans including gastro-Intestinal parasites such as Ancylostoma, Necator, Ascaris, Strongyloides, Trichinella, Toxocara, Capillaria, Trichuris, Enterobius and parasites which are found in the blood or other tissues and organs such as filiarial worms and the extra-intestinal stages of Strongyloides, Trichinella and Toxocara.

The compounds are also of value in treating ectoparasite infections including in particular arthropod ectoparasites such as fleas, ticks, mites, lice, blowfly and biting insects and migrating dipterous larvae which can affect cattle and horses.

The compounds are also insecticides active against household pests such as the cockroach, clothes moth, carpet beetle and the housefly as well as being useful against arthropod pests of stored grain and of agricultural plants such as spider mites, aphids, caterpillars and against migratory orthopterans such as locusts. We have discovered that certain compounds within the scope of this Invention have unexpectedly high potent activity against important arthropod parasites of cats and dogs.

The compounds of formula (I) may be administered as a formulation appropriate to the specific use envisaged and to the particular species of host animal being treated and the parasite or insect Involved. For use as an anthelmintic the compounds may be administered by injection, either subcutaneously or intramuscularly, alternatively they may be administered orally In the form of a capsule, bolus, tablet, chewable tablet or liquid drench, or they may be administered as a topical formulation or as an implant. For topical application dip, spray, powder, dust, pour-on, spot-on, jetting fluid, shampoos, collar, tag or harness may be used. Such formulations are prepared in a conventional manner in accordance with standard veterinary practice. Thus capsules, boluses or tablets may be prepared by mixing the active ingredient with a suitable finely divided diluent or carrier, additionally containing a disintegrating agent and/or binder such as starch, lactose, talc, or magnesium stearate. A drench formulation may be prepared by dispersing the active ingredient in an aqueous solution together with dispersing or wetting agents and injectable formulations may be prepared in the form of a sterile solution or emulsion. Pour-on or spot-on formulations may be prepared by dissolving the active ingredient in an acceptable liquid carrier vehicle, such as butyl digol, liquid paraffin or non-volatile ester with or without addition of a volatile component such as isopropanol. Alternatively, pour-on, spot-on or spray formulations can be prepared by encapsulation to leave a residue of active agent on the surface of the animal. These formulations will vary with regard to the weight of active compound depending on the species of host animal to be treated, the severity and type of infection and the body weight of the host. Generally for oral parenteral and pour-on administration a dose of from about 0.001 to 10mg per g of animal body weight given as a single dose or in divided doses for a period of from 1 to 5 days will be satisfactory but of course there can be instances where higher or lower dosage ranges are indicated and such are within the scope of this invention.

As an alternative the compounds may be administrated with the animal feedstuff and for this purpose a concentrated feed additive or premix may be prepared for mixing with the normal animal feed.

For use as an insecticide and for treating agricultural pests the compounds are applied as sprays, dusts, pour-on formulations, emulsions and the like in accordance with standard agricultural practice.

For human use the compounds are administered as a pharmaceutically acceptable formulation in accordance with normal medical practice.

The invention is illustrated by the following Examples, in which "avermectin B2" refers to an avermectin having an OH substituent at the 23-position and a single bond at the 22-23 position, "avermectin B1" refers to an avermectin having a double bond at the 22-23 position, "avermectin B1a" is as for avermectin B1 and having a sec- butyl substituent at the 25-position. An avermectin monosaccharide is an avermectin derivative having an alpha-oleandrosyl substituent at the 13-position, the avermectins themselves having a 4¹-(alpha-oleandrosyl)-alpha-oleandrosyl group at this position.

NMR spectra were measured using a Bruker 300MHz spectrometer. Mass spectra were measured on a VG Mark I 7070E mass spectrometer using a sample matrix of triethylene glycol with solid sodium chloride.

### EXAMPLE 1

### 5-Cyanomethylidene-25-cyclohexylavermectin B2

Tetra-n-butylammonium bromide (0.5g), dimethylcyanomethylphosphonate (3.5ml), 1,3-dimethyl-3,4,5,6-tetrahydro-2-(1H)-pyrimidinone (1 ml), methylene chloride (50ml), and a solution of sodium hydroxide (17g) in water (50ml) were stirred together rapidly at 20°C for 0.5 hours. Then the dichloromethane layer was separated and filtered through phase-separating paper into a flask containing the compound of Preparation 1 (0.5g). The resultant solution was stirred at 20°C for 0.5 hour. The mixture was evaporated in vacuo at 40°C and then dissolved in methylene chloride (50ml) and then washed with saturated ammonium chloride solution (50ml). The organic layer was separated and dried over sodium sulphate, then filtered and evaporated in vacuo. The residue was purified by chromatography on silica gel (50g). Elution was with a mixture of methylene chloride and methanol. The proportion of methanol was increased from 0 to 7% over 2 litres. 50ml fractions were collected and the product identified by thin-layer chromatography. (Rf=0.3 using a solvent system of 5% methanol in methylene chloride). Final purification was achieved by reverse-phase HPLC, using a C18 Zorbax (Trademark, Dupont) column (21mm x 25cm) eluting with a mixture of methanol and water (80:20) at a flowrate of 9mls per minute. 4.5ml fractions were collected and fractions 118 to 130 were combined and evaporated to yield a compound of formula (I), wherein R¹ is OH, R² is cyclohexyl, R³ is 4'-(alpha-L-oleandrosyl)-L-oleandrosyloxy, R⁴ is H, the 22,23 double-bond is absent, R⁶ and R⁷ are methyl, R¹² is CN and R¹³ is H. The compound has characteristic mass and NMR spectra:
- Mass spectrum (FAB):: 976 (MK+).
- NMR spectrum (300 MHz):: δ (CDCl₃) ppm:
6.1 (s,1H)} H-3 and H-5a.
6.12 (s,1H)}
1.6 (s,3H, H-4a).

### EXAMPLE 2

### 5-(Carbamoylmethylidene-25-cyclohexyl)avermectin B2

The product of Example 1 (250mg) was dissolved in methylene chloride (100ml) and manganese dioxide (1.15g) was added. The resulting suspension was stirred at 20°C for 1 day. Then a further 1g of manganese dioxide was added and stirring continued for 2 days. Then another 1g of manganese dioxide was added to the reaction mixture and stirring continued for 1 week. The mixture was then filtered through a pad of celite and the filtrate evaporated. The residue was purified by reverse-phase HPLC, using a C18 Zorbax (Trademark, Dupont) column (21mm x 25cm) eluting with a mixture of methanol and water (75:25) at a flowrate of 9mls/min. The relevant fractions were combined to yield a compound of formula (I), wherein R¹ is OH, R² is cyclohexyl, R³ is 4'-(alpha-L-olendrosyl)-L-oleandrosyloxy, R⁴ is H, the 22,23 double-bond is absent, R⁶ and R⁷ are methyl, R¹² is CONH₂ and R¹³ is H. The compound has characteristic mass and NMR spectra:
- Mass spectrum (FAB):: 994 (MK+).
- NMR spectrum (300 MHz):: δ (CDCl₃) ppm:
6.6 (s,1H)} H-3 and H-5a
6.2 (s,1H)}
1.6 (s,3H, H-4a).
6.2 (br.s. 1H)} NH₂ of
5.5 (br.s. 1 H)} carbamoyl

### EXAMPLE 3

### 5-Cyanomethylidene-22,23-dihydroavermectin B1a monosaccharide

By the method of Example 1 the title compound was prepared from the compound of Preparation 2. Purification was achieved by column chromatography on silica gel (30g) eluting with a mixture of ethyl acetate in methylene chloride. The proportion of ethyl acetate was increased from 0 to 40% over 2 litres. Final purification was achieved by reverse-phase HPLC, using a C-18 Zorbax (Trademark, Dupont) column (21mm x 25cm) eluting with a mixture of methanol and water (81:19) at a flowrate of 9mls/min. 9ml fractions were collected. Fractions 70 to 80 were combined to yield a compound of formula (I), wherein R¹ is H, R² is 2-butyl, R³ is L-oleandrosyloxy, R⁴ is H, the double bond is absent, R⁶ and R⁷ are methyl, R¹² is CN and R¹³ is H. The compound has characteristic mass and NMR spectra:
- Mass spectrum (FAB):: 790 (MK+).
- NMR spectrum (300MHz):: δ (CDCl₃) ppm:
6.1 (s,1H)} H-3 and H-5a.
6.12 (s,1H)}
1.55 (s,3H, H-4a).

### EXAMPLE 4

### 5-Methylidene-22,23-dihydroavermectin B1a monosaccharide

To a stirred solution of methyl triphenylphosphonium bromide (430mg) in dry tetrahydrofuran at 0°C under nitrogen was added a solution of butyl lithium in hexanes (1.6M, 0.75ml). The suspension was stirred for 15 minutes at 0°C, then the compound of Preparation 2 (140mg) in dry tetrahydrofuran (10ml) was added. The cooling bath was removed and the stirred mixture allowed to warm to 20°C. After stirring at this temperature for 1 hour the reaction mixture was added to saturated ammonium chloride solution (50ml) and extracted with methylene chloride. The organic layer was separated and dried over sodium sulphate, then filtered and evaporated. The residue was purified by reverse-phase HPLC. The column used by a C18 Zorbax (Trademark, Dupont), (21mm x 25cm), eluting with a mixture of methanol and water (86:14) at a flow rate of 9mls/min. 9ml fractions were taken and fractions 58 to 64 were combined and evaporated to yield the compound of formula (I) wherein R¹ is H, R² is 2-butyl, R³ is L-oleandrosyloxy, R⁴ is H, the double bond is absent, R⁶ and R⁷ are methyl, R¹² and R¹³ are H. The compound has characteristic mass and NMR spectra:
- Mass spectrum (FAB):: 749 (MNa+).
- NMR spectrum (300MHz):: δ (CDCl₃) ppm:
5.55 (br.s., 2H, H-5a).
1.9 (br.s., 3H, H-4a).

### EXAMPLE 5

### 5-Methylidene-25-cyclohexyl-22,23-dihydroavermectin B1 monosaccharide

By the method of Example 4 the title compound was prepared from the compound of Preparation 3. Purification was achieved by column chromatography on silica gel (150g); eluting with hexane, then methylene chloride, then ethyl acetate. Relevant fractions were combined to yield, on evaporation, 760mg of crude material. Further purification was achieved by reverse-phase HPLC on a Dynamax C-18 (Trademark, Rainin) column, (41.4mm x 25cm) eluting with a mixture of methanol and water (88:12) at a flowrate of 40mls/min. Fractions 29 to 34 were combined and evaporated to yield 139mg of a compound of formula (I) wherein R¹ is H, R² is cyclohexyl, R³ is L-oleandrosyloxy, R⁴ is H, the double bond is absent, R⁶ and R⁷ are methyl, R¹² and R¹³ are H. The compound has characteristic mass and NMR spectra:
- Mass spectrum (FAB):: 775 (MNa+).
- NMR spectrum (300MHz):: δ (CDCl₃) ppm:
5.57 (br.s., 2H, H-5a).
1.95 (br.s., 3H, H-4a).

### EXAMPLE 6

### 5-Methylidene-25-cyclohexylavermectin B2

By the method of Example 4, the title compound was prepared from the compound of Preparation 1. Following silica gel chromatography, 80mg of a crude product was isolated. This was further purified by reverse-phase HPLC on a Dynamax C-18 (Trademark, Rainin) column, (41.4mm x 25cm) eluting with a mixture of methanol and water (85:15) at a flowrate of 40mls/min. 30ml fractions were collected, and fractions 35 to 44 were combined and evaporated to yield the compound of formula (I), (130mg), wherein R¹ is OH, R² is cyclohexyl, R³ is 4'-(alpha-L-oleandrosyl)-L-oleandrosyloxy, R⁴ is H, the 22,23 double bond is absent, R⁶ and R⁷ are methyl and R¹² and R¹³ are H. The compound has characteristic mass and NMR spectra:
- Mass spectrum (FAB):: 930 (MNH₄+).
- NMR spectrum (300MHz):: δ (CDCl₃) ppm:
5.55 (br.s., 2H, H-5a).
1.9 (br.s., 3H, H-4a).

### EXAMPLE 7

### 5-Ethylidene-25-cyclohexylavermectin B2

The title compound was prepared by the method of Example 6, except that ethyl triphenylphosphonium bromide was used instead of methyl triphenylphosphonium bromide. The product was purified by reverse-phase HPLC using a C-18 Zorbax (Trademark, Dupont) column (21mm x 25cm) eluting with a mixture of methanol and water (84:16) at a flowrate of 9mls/min. 9ml fractions were collected and fractions 86 to 90 were combined and evaporated to yield a compound of formula (I) wherein R¹ is OH, R² is cyclohexyl, R³ is 4'-(alpha-L-oleandrosyl)-L-oleandrosyloxy, R⁴ is H, the 22,23 double bond is absent, R⁶ and R⁷ are methyl, R¹² is H and R¹³ is methyl. The compound has characteristic mass and NMR spectra:
- Mass spectrum (FAB):: 949 (MNa+).
- NMR spectrum (300MHz):: δ (CDCl₃) ppm:
5.55 (br.s., 1H, H-5a).
2.15 (br.s., 3H, H-5b).
2.0 (br.s., 3H, H-4a).

### PREPARATION 1

### 5-Keto-25-cyclohexylavermectin B2

To a suspension of manganese dioxide (3g) in diethyl ether (50ml) was added 25-cyclohexylavermectin B2 (2g), obtained as described in EP-A-214731. The mixture was stirred for 1 day, and a further portion of manganese dioxide (3g) was added. After stirring for a second day at 20°C a third portion of manganese dioxide was added and the mixture stirred for a third day. The manganese dioxide was then removed by filtration through Celite and the filtrate was evaporated to give 0.6g of the title compound which has characteristic mass and NMR spectra:
- Mass spectrum (FAB):: 937 (MNa+).
- NMR spectrum (300MHz):: δ (CDCl₃) ppm:
6.6 (br.s., 1H, H-3).
1.9 (br.s., 3H, H-4a).

### PREPARATION 2

### 5-Keto-22.23-dihydroavermectin B1a monosaccharide

By the method of Preparation 1, the title compound was prepared from 25-(2-butyl)-22,23-dihydroavermectin B1a monosaccharide obtained as described in US-4199569.

### PREPARATION 3

### 5-Keto-25-cyclohexyl-22,23-dihydroavermectin B1 monosaccharide

25-Cyclohexylavermectin B1 (9.9g) was dissolved in toluene (1 litre) and Wilkinson's catalyst (tristriphenylphosphine rhodium (I) chloride) (9.25g) was added. The solution was hydrogenated on a large Parr shaker at room temperature at 50 psi hydrogen pressure. After 3 hours the reaction vessel was depressurised and allowed to stand for 12 hours before addition of a further portion of catalyst (5g) and hydrogenated as before for a further 2 hours after which no starting material remained. The solution was filtered, evaporated to dryness under vacuum and the residue chromatographed on silica eluting with methylene chloride then methylene chloride:methanol 9:1. The crude product was then chromatographed again on silica (200g) eluting with methylene chloride:methanol 19:1 to give after evaporation of the solvent under vacuum impure 22,23-dihydro-25-cyclohexylavermectin B1 as a brown foam (10g). This material was dissolved in a mixture of isopropanol (200ml) and sulphuric acid (2ml) and the brown solution was stirred at room temeprature for 15 hours then poured into a mixture of ice and water (500ml) and extracted with methylene chloride (3 x 200ml). The organic layer was washed with saturated potassium hydrogen carbonate solution (100ml), water (2 x 50ml) dried over anhydrous magnesium sulphate and evaporated under vacuum to give a crude gum which was chromatographed on silica eluting with methylene chloride then methylene chloride:ethyl acetate 2:1 to give 22,23-dihydro-25-cyclohexylavermectin B1 monosaccharide. This compound was dissolved in anhydrous diethyl ether and the solution stirred with manganese dioxide to yield the title product.

## Claims

1. A compound of formula (I): 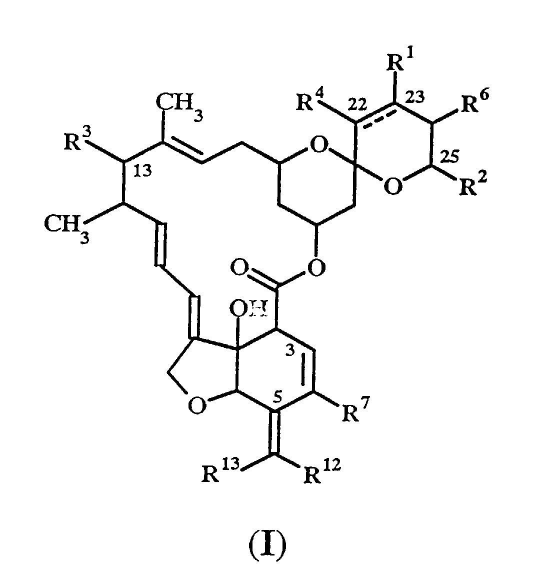 wherein the broken line represents an optional bond, R¹ and R⁴ being absent when this bond is present, R¹ and R⁴ are independently H or OR¹⁴ where R¹⁴ is H, C₁-C₈ alkyl, C₂-C₈ alkenyl, aralkyl, C₂-C₈ alkanoyl, C₃-C₈ alkenoyl, aralkanoyl, aroyl or carbamoyl;
R² is:
(a) an alpha-branched C₃-C₈ alkyl, alkenyl, alkoxy-alkyl, or alkylthioalkyl group; an alpha-branched C₄-C₈ alkynyl group; a (C₅-C₈)cycloalkyl-alkyl group wherein the alkyl group is an alpha-branched C₂-C₅ alkyl group; a C₃-C₈ cycloalkyl or C₅-C₈ Cycloalkenyl group, either of which may optionally be substituted by methylene or one or more C₁-C₄ alkyl groups or halo atoms; or a 3 to 6 membered oxygen or sulphur containing heterocyclic ring which may be saturated, or fully or partially unsaturated and which may optionally be substituted by one or more C₁-C₄ alkyl groups or halo atoms; or
(b) a group of the formula -CH₂R⁸ wherein R⁸ is H, C₁-C₈ alkyl, C₂-C₈ alkenyl, C₂-C₈ alkynyl, alkoxyalkyl or alkylthioalkyl containing from 1 to 6 carbon atoms in each alkyl or alkoxy group, wherein any of said alkyl, alkoxy, alkenyl or alkynyl groups may be substituted by one or more halo atoms; or R⁸ is a C₃-C₈ cycloalkyl or C₅-C₈ cycloalkenyl group, either of which may optionally be substituted by methylene or one or more C₁-C₄ alkyl groups or halo atoms; or R⁸ is a 3 to 6 membered oxygen or sulphur containing heterocyclic ring which may be saturated, or fully or partially unsaturated and which may optionally be substituted by one or more C₁-C₄ alkyl groups or halo atoms; or R⁸ is a group of the formula SR⁹ wherein R⁹ is C₁-C₈ alkyl, C₂-C₈ alkenyl, C₃-C₈ alkynyl, C₃-C₈ cycloalkyl, C₅-C₈ cycloalkenyl, phenyl or substituted phenyl wherein the substituent is C₁-C₄ alkyl, C₁-C₄ alkoxy or halo; or R⁸ is a 3 to 6 membered oxygen or sulphur containing heterocyclic ring which may be saturated, or fully or partially unsaturated and which may optionally be substituted by one or more C₁-C₄ alkyl groups or halo atoms; or
(c) a C₁-C₆ alkyl group substituted by one oxo or one or more hydroxy groups or by a single oxygen atom on two adjacent carbon atoms forming an oxirane ring, or R² is a C₁-C₅ alkyl group substituted by a (C₁-C₆) alkoxycarbonyl group, said substituents on R₂ being attached to either or both of a terminal carbon atom and a carbon atom adjacent a terminal carbon atom of R²; or
(d) = CH₂ or a group of the formula: 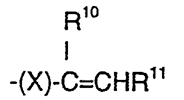 wherein R¹⁰ and R¹¹ are both H; R¹⁰ is H and R¹¹ is C₁-C₃ alkyl, or one of R¹⁰ and R¹¹ is H and the other is phenyl, heteroaryl, C₂-C₆ alkoxycarbonyl or substituted phenyl or heteroaryl wherein said substituent is fluorine, chlorine, C₁-C₄ alkyl, C₁-C₄ alkoxy, C₁-C₄ alkylthio, hydroxy(C₁-C₄)alkyl, cyano, aminosulphonyl, C₂-C₆ alkanoyl, C₂-C₆ alkoxycarbonyl, nitro, trifluoromethyl, trifluoromethoxy, amino or mono or di(C₁-C₄) alkylamino; and X is a direct bond or is an alkylene group having from 2 to 6 carbon atoms which may be straight or branched-chain; or
(e) phenyl which may optionally be substituted with at least one substituent selected from C₁-C₄ alkyl, C₁-C₄ alkylthio groups, halo atoms, trifluoromethyl, and cyano;
or R² may be a group of formula (II): 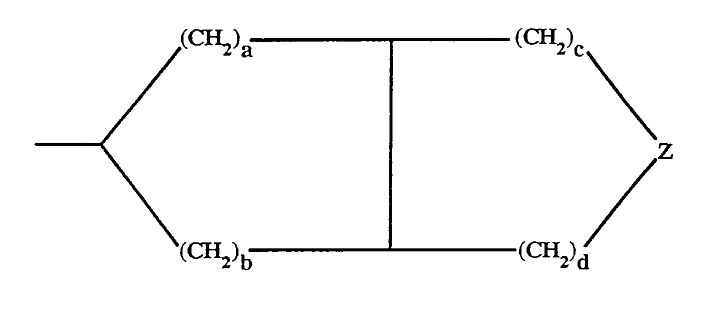 wherein Z is O, S or -CH₂- and a, b, c and d may each independently be 0, 1 or 2, the sum of a, b, c and d not exceeding 5;
R⁶ is H or C₁-C₆ alkyl;
R⁷ is CH₃, -CH₂-OR¹⁴ where R¹⁴ is as defined above, or -CH₂X where X is a halogen atom; and
R³ is a group of formula: 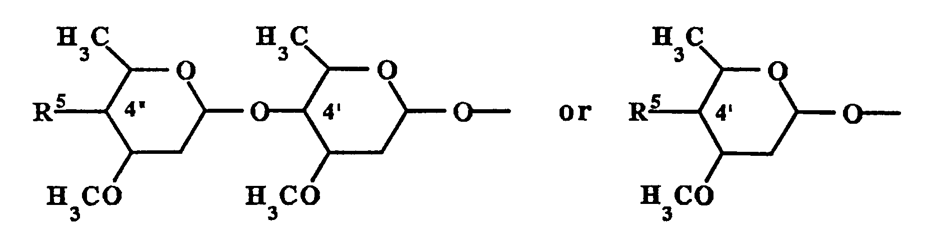 wherein R⁵ is attached to C-4" or C-4' by a single bond and is hydrogen, halo, hydroxy, C₁-C₉ alkanoyloxy or alkenoyloxy, aroyloxy, C₁-C₈ alkoxy, amino, N-(C₁-C₈)alkylamino, N,N-di(C₁-C₉)-alkylamino, N-(C₁-C₉)alkanoylamino, or N,N-di(C₁-C₉)alkanoylamino;
or R⁵ is attached to C-4" or C-4' by a double bond and is oxo, optionally substituted oximino, semicarbazido, N-(C₁-C₄)alkylsemicarbazono, N,N-di(C₁-C₄)alkylsemicarbazono, (C₁-C₄)alkylbenzoylhydrazono, and R¹² and R¹³ are independently H, CN, CONH₂, C₁-C₆ alkyl or aryl optionally substituted with at least one halo, OH, C₁-C₆ alkoxy or C₁-C₆ alkylthio group.

2. A compound according to claim 1, in which R⁴ is H and R¹ is OH.

3. A compound according to claim 1 in which R² is isopropyl, sec-butyl or cyclohexyl.

4. A compound according to any preceding claim, in which R⁵ is -OH.

5. A compound according to any preceding claim, in which one of R¹² and R¹³ is H and the other is H, methyl, cyano or carbamoyl.

6. A compound according to claim 1 which is:
5-cyanomethylidene-25-cyclohexyl avermectin B2;
5-carbamoylmethylidene-25-cyclohexyl avermectin B2;
5-cyanomethylidene-22,23-dihydroavermectin B1a monosaccharide;
5-methylidene-22,23-dihydroavermectin B1a monosaccharide;
5-methylidene-25-cyclohexyl-22,23-dihydroavermectin B1 monosaccharide;
5-methylidene-25-cyclohexylavermectin B2; or
5-ethylidene-25-cyclohexylavermectin 82.

7. A pharmaceutical or veterinary composition, comprising a compound according to any preceding claim and a pharmaceutically acceptable carrier or excipient.

8. A compound according to any one of claims 1 to 6, for use in animal or human medicine.

9. A compound according to any one of claims 1 to 6, for use as an antiparasitic agent.

10. Use of a compound according to any one of claims 1 to 6 for making a medicament for treatment or prophylaxis of parasite infestations.

11. A method of making a compound of formula (I) as defined in any one of claims 1 to 6 which comprises allowing a compound of formula (II), 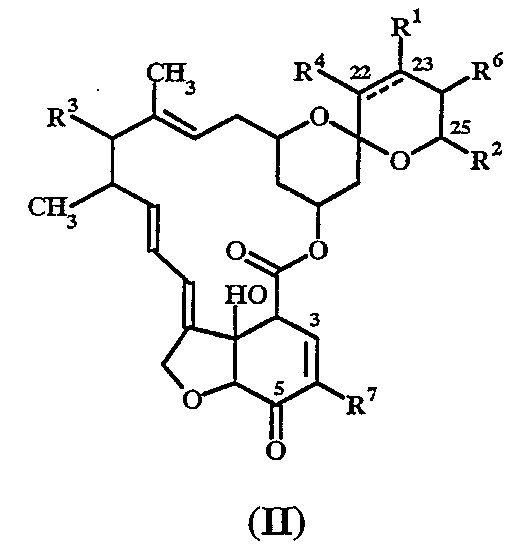 wherein R¹, R², R³, R⁴, R⁶ and R⁷ are as defined in any one of claims 1 to 5 to react with a compound of formula R¹³R¹²C=PPh₃ where R¹² and R¹³ are as defined in any one of claims 1 to 5 and Ph is phenyl.

12. A method according to claim 11, in which the reaction takes place in an inert organic solvent at a temperature from -100°C to 0°C.

13. A method according to claim 11 or 12 in which R¹² or R¹³ is cyano and the cyano group is subsequently converted to a carbamoyl group by hydrolysis.

## Patentansprüche

1. Verbindung der folgenden Formel I: 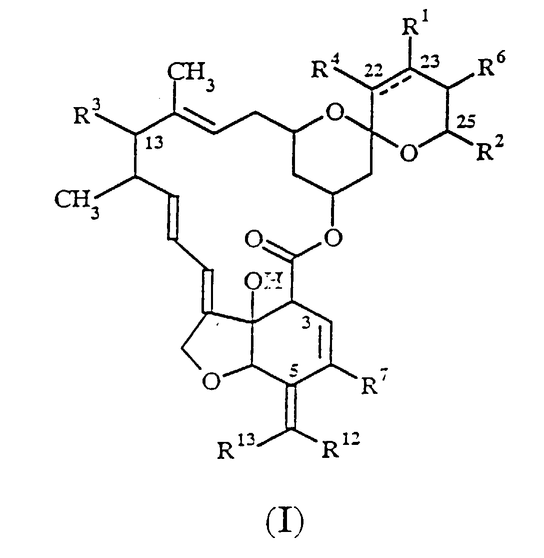 worin die gestrichelte Linie für eine optionale Bindung steht, R¹ und R⁴ fehlen, wenn diese Bindung vorhanden ist, R¹ und R⁴ unabhängig voneinander H oder OR¹⁴ mit R¹⁴ gleich H, C₁-C₈-Alkyl, C₂-C₈-Alkenyl, Aralkyl, C₂-C₈-Alkanoyl, C₃-C₈-Alkenoyl, Aralkanoyl, Aroyl oder Carbamoyl;
R²
(a) eine alphaverzweigte C₃-C₈-Alkylgruppe, Alkenylgruppe, Alkoxyalkylgruppe oder Alkylthioalkylgruppe; eine alphaverzweigte C₄-C₈-Alkinylgruppe; eine (C₅-C₈)Cycloalkyl-alkylgruppe, worin die Alkylgruppe für eine alphaverzweigte C₂-C₅-Alkylgruppe steht; eine C₃-C₈-Cycloalkylgruppe oder eine C₅-C₈-Cycloalkenylgruppe, wobei jede der beiden gegebenenfalls durch Methylen oder eine oder mehrere C₁-C₄-Alkylgruppen oder Halogenatome substituiert sein kann; oder einen 3- bis 6-gliedrigen, Sauerstoff oder Schwefel enthaltenden heterocyclischen Ring, der gesättigt oder vollständig oder teilweise ungesättigt sein kann und der gegebenenfalls durch eine oder mehrere C₁-C₄-Alkylgruppen oder Halogenatome substituiert sein kann; oder
(b) eine Gruppe der Formel -CH₂R⁸, worin R⁸ für H, C₁-C₈-Alkyl, C₂-C₈-Alkenyl, C₂-C₈-Alkinyl, Alkoxyalkyl oder Alkylthioalkyl mit 1 bis 6 Kohlenstoffatomen in jeder Alkyl- oder Alkoxygruppe, wobei jede der Alkyl-, Alkoxy-, Alkenyl- oder Alkinylgruppen durch ein oder mehrere Halogenatome substituiert sein kann, steht oder R⁸ für eine C₃-C₈-Cycloalkyl- oder C₅-C₈-Cycloalkenylgruppe, wobei jede dieser Gruppen gegebenenfalls durch Methylen oder eine oder mehrere C₁-C₄-Alkylgruppen oder Halogenatome substituiert sein kann, steht, oder R⁸ für eine Gruppe der Formel SR⁹, worin R⁹ C₁-C₈-Alkyl, C₂-C₈-Alkenyl, C₃-C₈-Alkinyl, C₃-C₈-Cycloalkyl, C₅-C₈-Cycloalkenyl, Phenyl oder substituiertes Phenyl mit dem Substituenten gleich C₁-C₄-Alkyl, C₁-C₄-Alkoxy oder Halogen bedeutet, oder R⁸ für einen 3- bis 6-gliedrigen, Sauerstoff oder Schwefel enthaltenden, heterocyclischen Ring, der gesättigt oder vollständig oder teilweise ungesättigt sein kann und der gegebenenfalls durch eine oder mehrere C₁-C₄-Alkylgruppen oder Halogenatome substituiert sein kann, steht; oder
(c) eine durch eine Oxogruppe oder eine oder mehr Hydroxygruppen oder ein einzelnes Sauerstoffatom an zwei benachbarten Kohlenstoffatomen unter Bildung eines Oxiranrings substituierte C₁-C₆-Alkylgruppe bedeutet oder R² eine C₁-C₅-Alkylgruppe, die durch eine C₁-C₆-Alkoxycarbonylgruppe substituiert ist, wobei die Substituenten an R² an ein terminales Kohlenstoffatom und/oder ein zu dem terminalen Kohlenstoffatom von R² benachbartes Kohlenstoffatom gebunden sind, darstellt oder
(d) = CH₂ oder eine Gruppe der Formel: 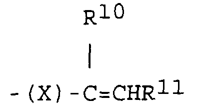 worin R¹⁰ und R¹¹ beide für H stehen, R¹⁰ H ist und R¹¹ C₁-C₃-Alkyl bedeutet oder einer der Reste R¹⁰ und R¹¹ für H steht und der andere Phenyl, Heteroaryl, C₂-C₆-Alkoxycarbonyl oder substituiertes Phenyl oder Heteroaryl, wobei der Substituent Fluor, Chlor, C₁-C₄-Alkyl, C₁-C₄-Alkoxy, C₁-C₄-Alkylthio, Hydroxy(C₁-C₄)Alkyl, Cyano, Aminosulfonyl, C₂-C₆-Alkanoyl, C₂-C₆-Alkoxycarbonyl, Nitro, Trifluormethyl, Trifluormethoxy, Amino oder Mono- oder Di(C₁-C₄)Alkylamino ist, bedeutet und X einer direkten Bindung entspricht oder eine Alkylengruppe mit 2 bis 6 Kohlenstoffatomen darstellt, die geradkettig oder verzweigt sein kann, oder
(e) Phenyl, das gegebenenfalls mit mindestens einem Substituenten, der unter C₁-C₄-Alkyl-, C₁-C₄-Alkylthiogruppen, Halogenatomen, Trifluormethyl und Cyano ausgewählt ist, substituiert ist, bedeutet oder R² eine Gruppe der folgenden Formel (II) sein kann: 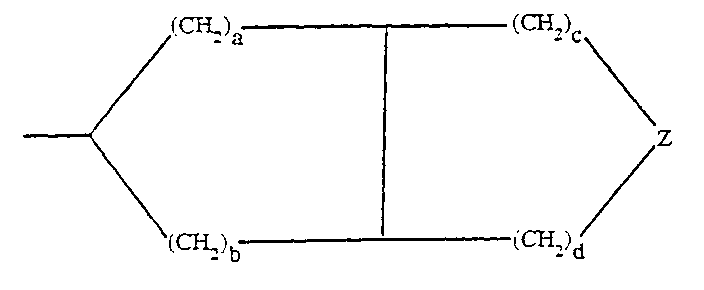 worin Z für O, S oder -CH₂- steht und a, b, c und d jeweils unabhängig voneinander 0, 1 oder 2 bedeuten können, wobei die Summe aus a, b, c und d 5 nicht überschreitet;
R₆ H oder C₁-C₆-Alkyl bedeutet und
R₇ CH₃, -CH₂-OR¹⁴ mit R¹⁴ in der oben angegebenen Bedeutung oder -CH₂X mit X gleich einem Halogenatom bedeutet und
R³ eine Gruppe der Formel 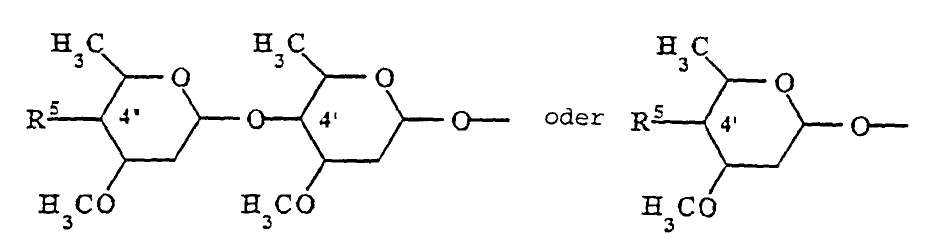 bedeutet, worin R⁵ über eine Einfachbindung an C-4" oder C-4' gebunden ist und für Wasserstoff, Halogen, Hydroxy, C₁-C₉-Alkanoyloxy oder -Alkenoyloxy, Aroyloxy, C₁-C₈-Alkoxy, Amino, N-(C₁-C₈)Alkylamino, N,N-Di(C₁-C₉)alkylamino, N-(C₁-C₅)Alkanoylamino oder N,N-Di(C₁-C₉)Alkanoylamino steht oder R⁵ über eine Doppelbindung an C-4" oder C-4' gebunden ist und für Oxo, gegebenenfalls substituiertes Oximino, Semicarbazido, N-(C₁-C₄)Alkylsemicarbazono, N,N-Di(C₁-C₄)alkylsemicarbazono oder (C₁-C₄)Alkylbenzoylhydrazono steht und R¹² und R¹³ unabhängig voneinander H, CN, CONH₂, C₁-C₆-Alkyl oder Aryl, gegebenenfalls substituiert durch mindestens ein(e) Halogenatom, OH-, C₁-C₆-Alkoxy- oder C₁-C₆-Alkylthiogruppe, bedeuten.

2. Verbindung nach Anspruch 1, worin R⁴ für H steht und R¹ OH bedeutet.

3. Verbindung nach Anspruch 1, worin R² Isopropyl, sec.- Butyl oder Cyclohexyl ist.

4. Verbindung nach einem der vorhergehenden Ansprüche, worin R⁵ für OH steht.

5. Verbindung nach einem der vorhergehenden Ansprüche, worin einer der Reste R¹² und R¹³ H ist und der andere H, Methyl, Cyano oder Carbamoyl bedeutet.

6. Verbindung nach Anspruch 1, nämlich:
5-Cyanomethyliden-25-cyclohexyl-avermectin-B2;
5-Carbamoylmethyliden-25-cyclohexyl-avermectin-B2;
5-Cyanomethyliden-22,23-dihydroavermectin-B1a-monosaccharid;
5-Methyliden-22,23-dihydroavermectin-B1a-monosaccharid;
5-Methyliden-25-cyclohexyl-22,23-dihydroavermectinB1-monosaccharid;
5-Methyliden-25 cyclohexylavarmectin-B2 und
5-Ethyliden-25 cyclohexylavarmectin-B2.

7. Pharmazeutische oder veterinärmedinische Zubereitung, die eine Verbindung nach einem der vorhergehenden Ansprüche und einen pharmazeutisch akzeptablen Träger oder ein pharmazeutisch akzeptables Streckmittel umfaßt.

8. Verbindung nach einem der Ansprüche 1 bis 6 zur Verwendung in der Tier- oder Humanmedizin.

9. Verbindung nach einem der Ansprüche 1 bis 6 zur Verwendung als antiparasitäres Mittel.

10. Verwendung einer Verbindung nach einem der Ansprüche 1 bis 6 zur Herstellung eines Medikaments zur Behandlung oder Prophylaxe von (bei) Parasitenbefall.

11. Verfahren zur Herstellung einer Verbindung der Formel (I) gemäß der Definition in einem der Ansprüche 1 bis 6 durch Umsetzen einer Verbindung der Formel (II) 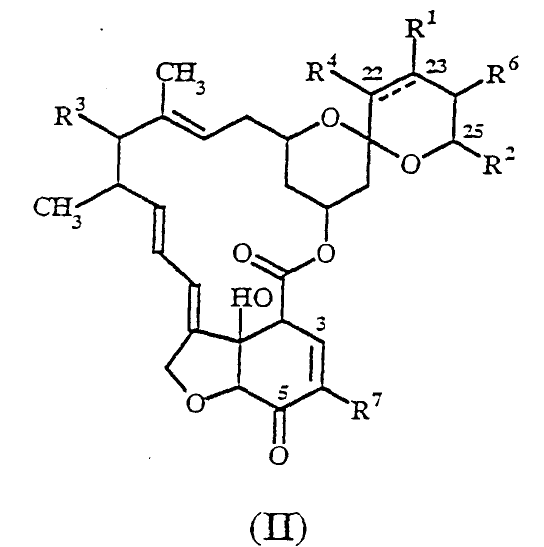
worin R¹, R², R³, R⁴, R⁶ und R⁷ die in einem der Ansprüche 1 bis 5 angegebene Bedeutung besitzen,
mit einer Verbindung der Formel R¹³R¹²C=PPh₃ mit R¹² und R¹³ in der in einem der Ansprüche 1 bis 5 angegebenen Bedeutung und Ph gleich Phenyl.

12. Verfahren nach Anspruch 11, worin die Reaktion in einem inerten organischen Lösungsmittel bei einer Temperatur von -100°C bis 0°C erfolgt.

13. Verfahren nach Anspruch 11 oder 12, worin R¹² oder R¹³ Cyano ist und die Cyanogruppe nachfolgend durch Hydrolyse in eine Carbamoylgruppe umgewandelt wird.

## Revendications

1. Composé de formule (I) : 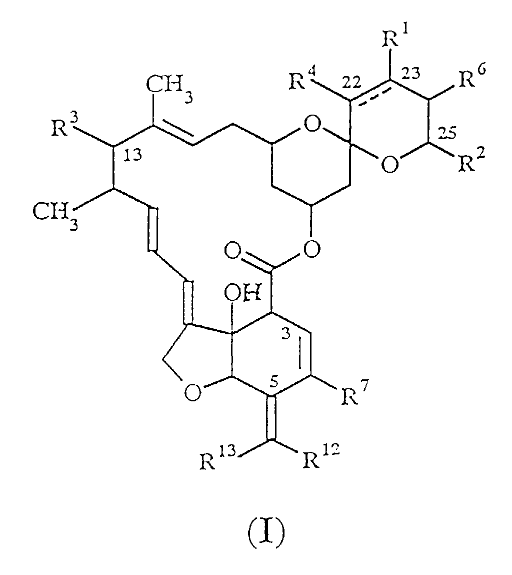 dans laquelle la ligne en pointillé représente une liaison facultative, R¹ et R⁴ sont absents lorsque cette liaison existe, R¹ et R⁴ représentent indépendamment H ou OR¹⁴ où R¹⁴ représente H, alkyle en C₁-C₈, alkényle en C₂-C₈, aralkyle, alcanoyle en C₂-C₈, alkénoyle en C₃-C₈, aralcanoyle, aroyle ou carmaboyle ;
R² représente :
(a) un groupe en C₃-C₈ ramifié en alpha, choisi parmi alkyle, alkényle, alcoxyalkyle, ou alkylthioalkyle ; un groupe alkynyle en C₄-C₈ ramifié en alpha ; un groupe (cycloalkyle en C₅-C₈)alkyle où le groupe alkyle est un groupe alkyle en C₂-C₅ ramifié en alpha ; un groupe cycloalkyle en C₃-C₈ ou cycloalkényle en C₅-C₈, dont l'un ou l'autre peut facultativement être substitué par un groupe méthylène ou un ou plusieurs groupes alkyle en C₁-C₄ ou atomes d'halogène ; ou un noyau hétérocyclique ayant de 3 à 6 chaînons et contenant de l'oxygène ou du soufre, lequel cycle peut être saturé ou totalement ou partiellement insaturé et peut facultativement être substitué par un ou plusieurs groupes alkyle en C₁-C₄ ou atomes d'halogène ; ou
(b) un groupe de formule -CH₂R⁸ dans lequel R⁸ représente H, alkyle en C₁-C₈, alkényle en C₂-C₈, alkynyle en C₂-C₈, alcoxyalkyle ou alkylthioalkyle contenant de 1 à 6 atomes de carbone dans chaque groupe alkyle ou alcoxy, l'un quelconque desdits groupes alkyle, alcoxy, alkényle ou alkynyle pouvant être substitué par un ou plusieurs atomes d'halogène ; ou R⁸ est un groupe cycloalkyle en C₃-C₈ ou cycloalkényle en C₅-C₈, l'un ou l'autre pouvant facultativement être substitué par un groupe méthylène ou par un ou plusieurs groupes alkyle en C₁-C₄ ou atomes d'halogène ; ou R⁸ est un noyau hétérocyclique ayant de 3 à 6 chaînons et contenant de l'oxygène ou du soufre, ledit cycle pouvant être saturé, ou totalement ou partiellement insaturé et pouvant être facultativement substitué par un ou plusieurs groupes alkyle en C₁-C₄ ou atomes d'halogène ; ou R⁸ est un groupe de formule SR⁹ où R⁹ est un groupe alkyle en C₁-C₈, alkényle en C₂-C₈, alkynyle en C₃-C₈, cycloalkyle en C₃-C₈, cycloalkényle en C₅-C₈, phényle ou phényle-substitué où le substituant est un groupe alkyle en C₁-C₄, alcoxy en C₁-C₄ ou halogéno ; ou R⁸ est un noyau hétérocyclique ayant de 3 à 6 chaînons contenant de l'oxygène ou du soufre et pouvant être saturé ou totalement ou partiellement insaturé et éventuellement substitué par un ou plusieurs groupes alkyle en C₁-C₄ ou atomes d'halogène ; ou
(c) un groupe alkyle en C₁-C₆ substitué par un groupe oxo ou un ou plusieurs groupes hydroxy ou par un atome d'oxygène unique sur deux atomes de carbone adjacents formant un noyau oxirane, ou R² est un groupe alkyle en C₁-C₅ substitué par un groupe (alcoxy en C₁-C₆)carbonyre, lesdits substituants sur R² étant fixés sur un atome de carbone terminal et un atome de carbone adjacent à un atome de carbone terminal de R² ou à l'un quelconque d'entre eux ; ou
(d) =CH₂ ou un groupe de formule : 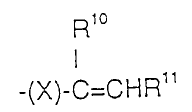 où R¹⁰ et R¹¹ représentent tous deux H ; R¹⁰ représente H et R¹¹ représente un groupe alkyle en C₁-C₃, ou l'un des radicaux R¹⁰ et R¹¹ représente H et l'autre représente phényle, hétéroaryle, (alcoxy en C₂-C₆) carbonyle ou phényle ou hétéroaryle substitués, ledit substituant étant un groupe fluoro, chloro, alkyle en C₁-C₄, alcoxy en C₁-C₄, (alkyle en C₁-C₄)thio, hydroxy(alkyle en C₁-C₄), cyano, aminosulfonyle, alcanoyle en C₂-C₆, (alcoxy en C₂-C₆)carbonyle, nitro, trifluorométhyle, trifluorométhoxy, amino ou mono- ou di(alkyle en C₁-C₄)amino ; et X est une liaison directe ou un groupe alkylène ayant de 2 à 6 atomes de carbone qui peut être à chaîne droite ou ramifiée ; ou
(e) phényle qui peut facultativement être substitué par au moins un substituant choisi parmi alkyle en C₁-C₄, (alkyle en C₁-C₄)thio, halogéno, trifluorométhyle, et cyano ;
ou R² peut être un groupe de formule (II) : 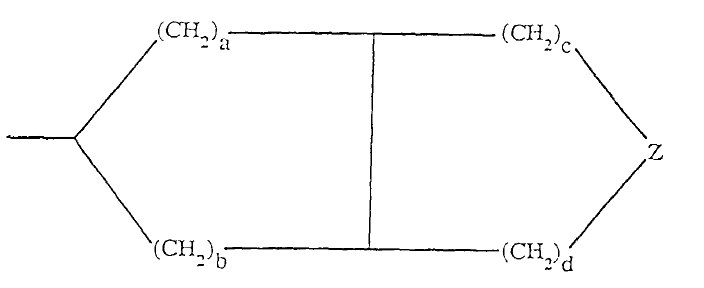 dans laquelle Z représente O, S ou -CH₂- et a, b, c et d peuvent être chacun indépendamment 0, 1 ou 2, la somme de a, b, c et d n'excédant pas 5 ;
R⁶ représente H ou alkyle en C₁-C₆ ;
R⁷ représente CH₃, -CH₂-OR¹⁴ où R¹⁴ est tel que défini plus haut, ou -CH₂X où X est un atome d'halogène ; et
R³ est un groupe de formule : 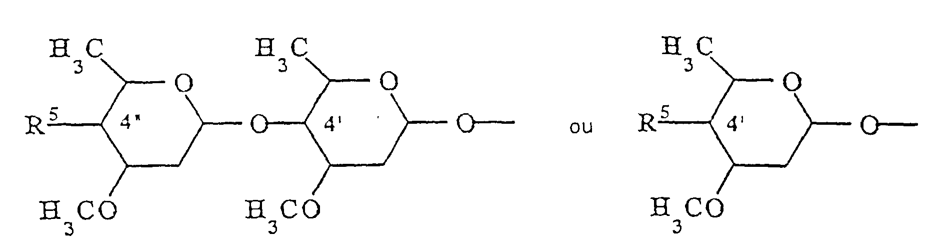
où R⁵ est fixé au carbone en position -4" ou -4' par une liaison simple et représente l'hydrogène, un groupe halogéno, hydroxy, (alcanoyle en C₁-C₉)oxy, ou (alkénoyle en C₁-C₉)oxy, aroyloxy, alcoxy en C₁-C₈, amino, N-(alkyle en C₁-C₈) amino, N,N-di(alkyle en C₁-C₉)amino, N-(alcanoyle en C₁-C₉)amino, ou N,N-di(alcanoyle en C₁-C₉)amino ;
ou R⁵ est fixé au carbone en position -4" ou -4' par une double liaison et représente oxo, oximino facultativement substitué, semicarbazido, N- (alkyle en C₁-C₄)semicarbazono, N,N-di(alkyle en C₁-C₄)semicarbazono, (alkyle en C₁-C₄)benzoylhydrazono, et R¹² et R¹³ représentent indépendamment H, CN, CONH₂, alkyle en C₁-C₆ ou aryle facultativement substitué par au moins un groupe halogéno, OH, alcoxy en C₁-C₆ ou (alkyle en C₁-C₆)thio.

2. Composé selon la revendication 1, dans lequel R⁴ représente H et R¹ représente OH.

3. Composé selon la revendication 1, dans lequel R² représente isopropyle, sec-butyle ou cyclohexyle.

4. Composé selon l'une quelconque des revendications précédentes, dans lequel R⁵ représente -OH.

5. Composé selon l'une quelconque des revendications précédentes, dans lequel l'un des radicaux R¹² et R¹³ représente H et l'autre représente H, méthyle, cyano ou carbamoyle.

6. Composé selon la revendication 1, qui est la :
5-cyanométhylidène-25-cyclohexylavermectine B2 ;
5-carbamoylméthylidène-25-cyclohexylavermectine B2 ;
5-cyanométhylidène-22,23-dihydroavermectine B1a monosaccharide ;
5-méthylidène-22,23-dihydroavermectine B1a monosaccharide ;
5-méthylidène-25-cyclohexyl-22,23-dihydroavermectine B1 monosaccharide ;
5-méthylidène-25-cyclohexylavermectine B2 ; ou
5-éthylidène-25-cyclohexylavermectine B2.

7. Composition pharmaceutique ou vétérinaire comprenant un composé selon l'une quelconque des revendications précédentes et un excipient ou un véhicule pharmaceutiquement acceptable.

8. Composé selon l'une quelconque des revendications 1 à 6, pour une utilisation en médecine humaine ou vétérinaire.

9. Composé selon l'une quelconque des revendications 1 à 6, pour une utilisation comme agent antiparasitaire.

10. Utilisation d'un composé selon l'une quelconque des revendications 1 à 6, pour la fabrication d'un médicament pour le traitement ou la prophylaxie d'infestation parasitaire.

11. Procédé de fabrication d'un composé de formule (I) tel que défini dans l'une quelconque des revendications 1 à 6, qui consiste à mettre en contact réactionnel un composé de formule (II). 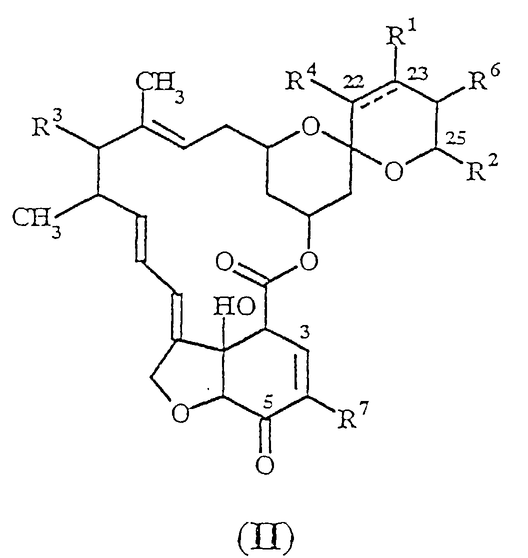 dans laquelle R¹, R², R³, R⁴, R⁶ et R⁷ sont tels que définis dans l'une quelconque des revendications 1 à 5, avec un composé de formule R¹³R¹²C=PPh₃ dans laquelle R¹² et R¹³ sont tels que définis dans l'une quelconque des revendications 1 à 5 et Ph représente phényle inerte à une température comprise entre -100°C et 0°C..

12. Procédé selon la revendication 11, dans lequel la réaction est mise en oeuvre dans un solvant organique.

13. Procédé selon la revendication 11 ou 12, dans lequel R¹² ou R¹³ représentent cyano et le groupe cyano est ultérieurement transformé en groupe carbamoyle par hydrolyse.
